# EUROPEAN PATENT SPECIFICATION

(11) **EP 1 069 824 B1**
(45) Date of publication and mention of the grant of the patent: **17.09.2003**
(21) Application number: 99912025.6
(22) Date of filing: 25.03.1999
(51) Int. Cl.: A01N 31/02, A01N 25/04, A61K 7/16, A61L 9/01, A23G 3/00, A23G 3/30, A61C 15/02, A61C 15/04, A24B 15/30

(54) **ORAL ANTIMICROBIAL AND ANTI-ODOUR COMPOSITIONS**
ORALE ANTIMIKROBIELLE UND DESODORIENDE ZUSAMMENSETZUNGEN
COMPOSITIONS ORALES ANTIMICROBIENNES ET ANTI-ODEURS

(30) Priority: 06.04.1998 IL 12396598
(43) Date of publication of application: 24.01.2001
(73) Proprietor: Innoscent Ltd., 52656 Ramat-Gan (IL)
(72) Inventor: ROSENBERG NEVO, Melvyn, 52596 Ramat-Gan (IL)
(74) Representative: Hay, Martin Alexander
(86) International application number: IL9900171
(87) International publication number: WO99051093

(56) References cited:
- EP-A- 0 129 778
- EP-A- 0 208 403
- WO-A-96/37183
- WO-A-97/13495
- US-A- 4 340 628
- US-A- 5 016 655
- US-A- 5 091 111
- CHEMICAL ABSTRACTS, vol. 28, no. 4, 20 February 1934 Columbus, Ohio, US; abstract no. 1099 3, DAVID I. MACHT & MARY E. DAVIS: "Local anesthetic properties of some aliphatic alcohols" column 1099; XP002074249 & PROCEEDINGS OF THE SOCIETY FOR EXPERIMENTAL BIOLOGY & MEDICINE., vol. 30, 1933, pages 1294-5, NEW YORK US
- PATENT ABSTRACTS OF JAPAN vol. 12, no. 96 (C-484), 29 March 1988 & JP 62 230712 A (NICHIBAI BOEKI KK), 9 October 1987
- CHEMICAL ABSTRACTS, vol. 109, no. 10, 5 September 1988 Columbus, Ohio, US; abstract no. 79000, MIYAZAKI, TAKASHI ET AL: "Deodorant preparation" XP002074282 & JP 63 068169 A (NOK CORP., JAPAN)
- CHEMICAL ABSTRACTS, vol. 83, no. 18, 3 November 1975 Columbus, Ohio, US; abstract no. 152407, SATO, TORAO: "Deodorant and disinfectant" XP002074283 & JP 50 088236 A (HYODO, AKIO, JAPAN)
- HERMAN GERSHON & LARRY SHANKS: "Antifungal Properties of n-Alkanols, alpha,omega-n-Alkanediols, and omega-Chloro-alpha alkanols" JOURNAL OF PHARMACEUTICAL SCIENCES., vol. 69, no. 4, April 1980, pages 381-4, XP002074319 WASHINGTON US cited in the application
- CHEMICAL ABSTRACTS, vol. 93, no. 17, 27 October 1980 Columbus, Ohio, US; abstract no. 161851, KATO, NOBUYKI ET AL: "The antimicrobial characteristics of 1- alkanols" XP002074350 & BOKIN BOBAI (1980), 8(8), 325-31 CODEN: BOBODP;ISSN: 0385-5201,1980, cited in the application
- CHEMICAL ABSTRACTS, vol. 89, no. 11, 11 September 1978 Columbus, Ohio, US; abstract no. 85228, YASUDA-YASAKI, YOKO ET AL: "Inhibition of germination of Bacillus subtilis spores by alcohols" XP002074351 & SPORES (1978), 7, 113-16 CODEN: SPORAI;ISSN: 0584-9144,1978, cited in the application
- DATABASE WPI Section Ch, Week 8413 Derwent Publications Ltd., London, GB; Class B05, AN 84-078371 XP002107993 & JP 59 029619 A (HASEGAWA CO LTD) , 16 February 1984
- DATABASE WPI Section Ch, Week 9334 Derwent Publications Ltd., London, GB; Class D22, AN 93-269079 XP002108101 & JP 05 184649 A (MORISHITA JINTAN KK) , 27 July 1993

## Description

### Field of the Invention

The present invention is concerned with compositions containing higher alcohols, particularly those having chain length C9 and greater, and their formulation for use as antimicrobial and anti-odor agents, particularly for oral use.

### Background of the Invention

Bacterial products and activity are implicated in many oral diseases, such as periodontal disease and caries, as well as in the production of unpleasant odors in the mouth. Because of the multitude of bacterial species found in the oral cavity, there is a need for effective non-specific anti-microbial agents, with a broad spectrum of activity. Such agents may be incorporated into a number of different preparations, including: mouthwashes, mouthsprays, toothpastes and other dentifrices, chewing gum, candies and related products.

For many years, short-chain alkyl alcohols such as ethanol and 2-propanol have been used clinically and domestically as surface-active anti-microbial agents. In addition, there have been a few reports that describe inhibitory effects of individual higher alcohols (chain length up to C16) on the growth of certain bacteria (particularly gram positive species), bacterial spores and fungi [see Kato & Shibasaki, *Bokin Bobai*.8: 325 (1980); Gershon & Shanks, *J. Pharm. Sci. 69: 381 (1980)*; Yasuda-Yasaki *et al., Spores 7*: 113 (1978); European patent application publication number 0208403].

In addition, JP 62-230712 (assigned to Nichibai Boeki KK) discloses the use of higher alcohols in the management of dental caries, a condition in which the gram positive bacteria *Streptococcus mutants* is believed to play a major pathogenic role. [See also JP 59 029619 (assigned to Hasegawa Co Ltd)]

US 4,340,628 discloses the use of linoleyl alcohol and linolenyl alcohol in the treatment of dental caries and peridontal diseases.

Compositions comprising higher alcohols are also known for various other purposes, as described in US 5 091 111; Macht D.I. and Davis, M. E., Proceedings of the Society for Experimental Biology and Medicine., Vol 30, 1933, pages 1294-5; EP-A-0129778; Chemical Abstracts, Vol. 109, No. 10; 5 September 1988, abstract no. 79000 (JP 63 068169) and Chemical Abstracts, Vol. 83, No. 18, 3 November 1975, abstract no. 152407 (JP 50 088236).

WO 97/13495 discloses antimicrobial compositions comprising a (C3-C6) alcohol and an antimicrobially effective amount of thymol and one or more other essential oils for the prevention and elimination of bad breath and for the reduction of oral microorganisms responsible for the development of dental plaque, gingivitis and tooth decay.

US 5 016 655 discloses the use in cigarettes of certain alcohols as inhibitors of the selective localization of nitrosamines in the tissues of a smoker.

JP 05 184649 discloses deodorant compositions containing peppermint and lemon oils.

WO 96/37183 discloses compositions capable of masking astringent taste sensations, which comprise a humectant component and a salt.

The current invention concerns higher alcohols (1-nonanol, 1-decanol and 1-undecanol), used singly or in combinations, and presented in a vehicle consisting either of a lower alcohol or a multiphase oil-aqueous preparation, which are highly effective as antimicrobial agents against a very broad spectrum of bacteria and yeasts. The invention also provides the formulation and use of these higher alcohols as anti-odor agents. While the mechanism of the anti-odor effect is not precisely known, it may be related to a shift in microbial populations at the treatment site. It would appear that it is particularly the gram negative resident species of the oral cavity that are implicated in the production of bad breath. The hitherto unreported highly efficient inhibition of these species by the higher alcohols is thus one of the key features of this invention.

In addition, the present invention also provides a unique two-phase solvent system for the sustained delivery of the anti-odor or anti-microbial alcohols at their site of action, particularly in the oral cavity.

The higher alcohols have not previously been used as the primary components of anti-odor products for oral use. This may be, in part, because of their characteristic, strong, citrus-like odors and flavors. In the present invention, however, it has been surprisingly found that low concentrations of the higher alcohols have a pleasant flavor and odor. Thus, when used at low concentrations, the higher alcohols, in addition to their primary antimicrobial and anti-odor effects, also confer positive organoleptic properties on oral anti-odor agents containing said higher alcohols. In another aspect of the invention, the abovementioned problematic strong odors and flavors contributed by the higher alcohols when used at higher concentrations is overcome by the use of novel combinations of taste-masking additives.

It is a primary purpose of this invention to provide the use of compositions containing higher alcohols as oral anti-odor preparations.

It is another purpose of this invention to provide oral anti-odor compositions containing higher alcohols together with taste-masking additives.

It is yet another purpose of this invention to provide the aforementioned oral anti-odor compositions in a variety of forms including mouthwash, toothpaste and candies.

It is a further purpose of this invention to provide an antimicrobial composition comprising a higher alcohol together with a lower alcohol.

It is a further purpose of this invention to provide an antimicrobial composition comprising a higher alcohol together with a multi-phase oil-aqueous vehicle.

Other objects and advantages of the invention will become apparent as the description proceeds.

### SUMMARY OF THE INVENTION

It has now been surprisingly found, and this is an object of the invention, that the higher alkyl alcohols are highly effective as oral anti-odor agents, displaying an unexpectedly high level of activity against gram negative bacteria, which have been reported to be the main causative agents in the development of bad breath. This stands in sharp contrast with what has been disclosed in the prior art, where the higher alcohols are described as possessing antibacterial effects primarily on gram positive organisms.

The invention is primarily directed to the use of a composition comprising a higher alcohol together with taste-masking additives, as an oral anti-odor preparation. Particularly, the invention is directed to the use of a composition comprising a higher alcohol selected from 1-nonanol, 1-decanol and 1-undecanol, or mixtures thereof and taste-masking additives, as an oral anti-odor preparation.

According to one preferred embodiment of the invention, the higher alcohol is 1-nonanol. According to another preferred embodiment, the higher alcohol used 1-decanol. In another preferred embodiment, the higher alcohol is 1-undecanoL

Although any suitable taste-masking additives may be used in conjunction with the above-mentioned higher alcohols, preferred taste-masking additives include nerol, citral and peppermint oil, or mixtures thereof.

In another aspect, the invention is directed to an anti-odor toothpaste comprising a higher alcohol selected from 1-nonanol, 1-decanol and 1-undecanol, or mixtures thereof, together with one or more taste-masking additives.

According to one preferred embodiment of the invention, the higher alcohol is 1-nonanol. According to another preferred embodiment, the higher alcohol used 1-decanol. In another preferred embodiment, the higher alcohol is 1-undecanol.

Although any suitable taste-masking additives may be used in conjunction with the above-mentioned higher alcohols, preferred taste-masking additives include nerol, citral and peppermint oil, or mixtures thereof.

In a further aspect, the invention is directed to an anti-odor candy comprising a higher alcohol selected from 1-nonanol, 1-decanol and 1-undecanol, or mixtures thereof, together with one or more taste-masking additives.

The above-mentioned anti-odor candy may be in the form of any of the commonly found types of confection. According to one preferred embodiment, the anti-odor candy is a chocolate candy. In another preferred embodiment the anti-odor candy is in the form of a chewable candy. In a further preferred embodiment, the anti-odor candy is in the form of chewing gum.

In some instances, it is desirable to provide the active compositions of the invention in sequestered form, so that they are delivered to the appropriate site in the oral cavity, and at the desired time. Illustrative examples of suitable sequestration methods include, e.g., microencapsulation, dispersion in a solid matrix, etc. Many such methods are known in the art, and will be apparent to the skilled person.

One such example is a dragé-covered chewing gum, which incorporates the active composition of the invention in the gum itself. Typically, the dragé will not comprise the composition of the invention, which will be liberated in to the oral cavity by the chewing action.

In another preferred embodiment, the anti-odor candy of the invention is a slowly dissolving product.

While the slowly dissolving candy may take many forms, in a preferred embodiment, it is a pastille. According to another preferred embodiment, the slowly dissolving product is a hard candy.

All of the abovementioned anti-odor candies may comprise any of the aforementioned higher alcohols. According to a preferred embodiment, however, the higher alcohol is 1-nonanol. According to another preferred embodiment, the higher alcohol is 1-decanol. In a further preferred embodiment, the higher alcohol is 1-undecanol. Although each of the above-mentioned anti-odor candies may contain any suitable taste-masking additives, in a preferred embodiment, the taste-masking additives comprise nerol, citral and peppermint oil.

In another aspect, the invention is directed to an anti-odor mouthwash comprising a higher alcohol selected from 1-nonanol, 1-decanol and 1-undecanol, or mixtures thereof, together with taste-masking additives. According to one preferred embodiment of the invention, the higher alcohol is 1-nonanol. According to another preferred embodiment, the higher alcohol used 1-decanol. In another preferred embodiment, the higher alcohol is 1-undecanol.

Although the anti-odor mouthwash may contain any suitable taste-masking additives, preferred taste-masking additives include nerol, citral and peppermint oil, or mixtures thereof.

The invention also encompasses an anti-odor mouthspray comprising a higher alcohol selected from 1-nonanol, 1-decanol and 1-undecanol, or mixtures thereof, together with taste-masking additives.

In a further aspect, the invention is directed to an anti-odor cigarette, comprising a higher alcohol selected from 1-nonanol, 1-decanol and 1-undecanol, or mixtures thereof.

The invention further makes provision for a toothpick that is coated or impregnated with a composition comprising a higher alcohol selected from among 1-nonanol, 1-decanol and 1-undecanol, or mixtures thereof, together with taste-masking additives.

In a further aspect, the invention is directed to a dental floss yarn that is coated or impregnated with a composition comprising a higher alcohol selected from among 1-nonanol, 1-decanol and 1-undecanol, or mixtures thereof, together with taste-masking additives.

In a further aspect, the invention is directed to use of an antimicrobially-effective amount of a higher alcohol selected from among 1-nonanol, 1-decanol and 1-undecanol, or mixtures thereof, in a (C1-C4) alcohol or multiphase oil-aqueous vehicle in the preparation of an antimicrobial composition suitable for oral use.

While there is no clear-cut definition of a "lower alcohol", alcohols containing up to 4 carbon atoms (C1-C4) are termed "lower alcohols" herein, and the alcohols 1-nonanol, 1-decanol and 1-undecanol are regarded as "higher alcohols", and this is the terminology adopted herein.

In another aspect, the invention is directed to the use of a higher alcohol selected from among 1-nonanol, 1-decanol and 1-undecanol, or mixtures thereof, as an oral anti-odor agent.

According to a preferred embodiment of'the invention, the higher alcohol is incorporated into a two-phase aqueous-oil system. In one aspect of the invention, this two-phase mixture is to be used in a pump-spray for reducing breath odor.

In another aspect, the invention is directed to the use of the above-mentioned higher alcohols or their mixtures as wound cleansing agents, and wherein the higher alcohol is dissolved in a (C1-C4) alcohol medium.

The invention also provides the use of these higher alcohols or mixtures thereof, for the removal or prevention of odors. In a preferred embodiment, the invention provides for the removal or prevention of odors, when said odors are present in the oral cavity.

In a further aspect, the invention is directed to a use of a composition comprising a lower alcohol and, as an additive, a higher alcohol selected from among 1-nonanol, 1-decanol and 1-undecanol, or mixtures thereof for the manufacture of a synergistic antimicrobial composition suitable for oral use. Lower alcohols are typically selected from among ethanol, methanol and 2-propanol. Preferred synergistic amounts of the higher alcohol are typically - but not limitatively - in the range of about 0.004% to 0.5%.

In a preferred embodiment, the composition contains ethanol as the lower alcohol.

While the above-mentioned synergistic anti-microbial composition may be provided in many forms, for many intended uses, according to a preferred embodiment of the invention, the composition is an anti-odor oral anti-microbial composition. According to another preferred embodiment, the synergistic anti-microbial composition is a wound-cleansing preparation, wherein the higher alcohol is dissolved in a lower alcohol medium.

In a further aspect, the invention is directed to a method for killing microorganisms, which comprises bringing the microorganisms to be killed into contact with a composition comprising an antimicrobially-effective amount of a higher alcohol selected from among 1-nonanol, 1-decanol and 1-undecanol, or mixtures thereof, dissolved in a lower alcohol medium. Preferably, the concentration of the higher alcohol is about 0.004% to 0.5%.

According to a preferred embodiment of the invention, the lower alcohol is selected from among ethanol, methanol and 2-propanol, or their mixtures. In a particularly preferred embodiment, the lower alcohol is ethanol.

The invention further encompasses a method for increasing the anti-microbial activity of a lower alcohol, or of a mixture of two or more lower alcohols, comprising adding to said lower alcohol or mixture of lower alcohols, a synergistic amount of a higher alcohol, wherein the higher alcohol is selected from among 1-nonanol, 1-decanol and 1-undecanol, or mixtures thereof.

In addition, the invention is further directed to the use of a composition comprising a higher alcohol selected from among 1-nonanol, 1-decanol and 1-undecanol, or mixtures thereof in a lower alcohol or multi-phase oil-aqueous vehicle, for the manufacture of an antimicrobial composition.

The invention also provides the use of a composition comprising a higher alcohol selected from 1-nonanol, 1-decanol and 1-undecanol, or mixtures thereof for inhibiting or destroying oral gram positive and gram negative bacteria.

In another aspect, the invention relates to an anti-odor composition adapted be brought into contact with the oro-pharynx and posterior portion of the oral cavity, comprising a higher alcohol selected from 1-nonanol, 1-decanol and 1-undecanol, or mixtures thereof, together with one or more taste-masking additives.

The invention further provides an anti-odor paste adapted to be applied to the tongue and to the teeth, comprising a higher alcohol selected from 1-nonanol, 1-decanol and 1-undecanol, or mixtures thereof, together with one or more taste-masking additives.

The invention also provides an anti-odor paste adapted to be supplied to the teeth and to be gargled, comprising a higher alcohol selected from 1-nonanol, 1-decanol and 1-undecanol, or mixtures thereof, together with one or more taste-masking additives.

All the above and other characteristics and advantages of the invention will be further understood from the following illustrative and non-limitative examples of preferred embodiments thereof.

### Detailed Description of Preferred Embodiments

For purposes of clarity and as an aid in the understanding of the invention, as disclosed and claimed herein, the following terms and abbreviations are defined below:
- **Synergistic amounts** -: refers to a small percentage of a higher alcohol which substantially improves the antimicrobial activity of the lower alcohol to which it is added.
- **Additive amounts** -: indicates amounts not exceeding 5 % of the total volume.
- **C*n* alcohols** -: indicates alkanols, where n is the number of carbon atoms.

### Examples

The following compositions and preparations were used in the examples given hereinbelow:

| 1. "Breathanol" | |
|---|---|
| nerol | 1 part |
| citral | 1 part |
| 1-nonanol | 1 part |
| peppermint oil | 2 parts |

| 2. Decarboxylase Medium | |
|---|---|
| peptone | 0.5 % |
| yeast extract | 0.3 % |
| dextrose | 0.1 % |
| bromocresol purple | 0.07 % |

### Example 1

### Inhibition of in vitro odor formation by toothpaste containing Breathanol

A series of test tubes were prepared, each containing:
a) 200 µl of freshly-taken morning saliva;
b) 10 - 1000 µl of a Breathanol-containing toothpaste that was diluted 1:5 with decarboxylase medium (concentration of Breathanol in the toothpaste *before* dilution is 1 %), and
c) decarboxylase medium, to bring the final volume of the mixture up to 5 ml.

The tubes were incubated at 37° C for three days. Following incubation, the odor was determined by a panel of odor judges, using the following semi-quantitative scale
0 = no odor
1= very slight odor
2 = weak but noticeable odor
3 = medium odor
4 = foul odor
5 = extremely foul odor

The results (Table I) show that a reduction in odor formation is seen with all dilutions of the Breathanol-containing toothpaste (compared with no-toothpaste control), and that this anti-odor effect is dose related.

**Table I**

| V**olume of diluted toothpaste (µl)** | **1-nonanolconcentration** | | **ODOR SCORES** | | | |
|---|---|---|---|---|---|---|
| | **ppm** | **% w/w** | **Judge 1** | **Judge 2** | **Judge 3** | **Mean** |
| 10 | 0.8 | 0.00008 | 3 | 4 | 1.25 | 2.75 |
| 25 | 2 | 0.0002 | 2 | 3 | 1.5 | 2.17 |
| 50 | 4 | 0.0004 | 2 | 2.5 | 0.5 | 1.67 |
| 125 | 10 | 0.001 | 1 | 1 | 1 | 1 |
| 250 | 20 | 0.002 | 0.5 | 1 | 0 | 0.5 |
| 500 | 40 | 0.004 | 0 | 1 | 0.75 | 0.58 |
| 750 | 60 | 0.006 | 0 | 1 | 1 | 0.67 |
| 1000 | 80 | 0.008 | 0 | 0 | 0.5 | 0.17 |
| Control | 0 | 0 | 4 | 4.5 | 4 | 4.17 |

### Example 2

### Clinical study of inhibition of odor formation by mouthwash containing Breathanol

The ability of Breathanol to reduce odor was tested in the following clinical study. Subjects (N = 51; mean age 24.5 years) were recruited from among those who had previously volunteered for similar studies. Subjects were remunerated for their time. The criteria for exclusion from the study were: taking antibiotics within one month prior to the study, smokers, partial or complete denture wearers. Participants were asked to refrain from eating or drinking 2 hours prior to measurements. Initially, subjects were tested for malodor-related parameters (as described further). They were then given the mouthwash (17 ml; active, containing 1 % Breathanol, or placebo), and were asked to swish and gargle twice for 30 seconds with a one minute interval. They were reexamined 1.5 and 3 hours following use.

Subjects were assessed for oral malodor-related parameters, including (i) whole mouth odor as measured by three independent judges on a scale of 0 - 5; (ii) tongue dorsum posterior odor using the spoon test; and (iii) volatile sulphide levels using the model 1170 sulphide monitor (Interscan Corp., Chatsworth CA).

All measurements were made prior to rinsing (time zero), and at 1.5 and 3 hours post-rinsing. Results were analyzed using analysis of variance (ANOVA) and, when necessary, analysis of co-variance (ANCOVA) with time zero as covariate.

### Measurements:

### Organoleptic measurements.

Organoleptic measurements were carried out throughout the study by one experienced and two inexperienced judges whose scores have been compared to other judge's scores and measurement techniques in previous studies.

Organoleptic measurements were made, based on the whole mouth expirate, as well as odor assessment from the posterior of the tongue dorsum. For whole mouth malodor, following a three hour fast, subjects were instructed to exhale briefly through the mouth, at a distance of ca. 10 cm from the nose of the judge. For assessment of the tongue posterior dorsum, a sample was obtained by mild scraping with a plastic spoon. After 5 seconds, the odor judges and the subjects themselves smelled the odor at a distance of ca. 5 cm from the spoon. Results of the two malodor assessments were rated on a semi-integer scale of -5 to 5 as follows:
-5 extremely pleasant odor
-4 very pleasant odor
-3 moderately pleasant odor
-2 slight, but noticeable pleasant odor
-1 barely noticeable pleasant odor
0 no appreciable odor
1 barely noticeable unpleasant odor
2 slight, but clearly noticeable unpleasant odor
3 moderate unpleasant odor
4 strong unpleasant odor
5 extremely foul odor

### Volatile sulphur compounds (VSC)

VSC of intraoral headspace was measured using the Interscan 1170 monitor. Quantitative measurement of volatile sulphides were carried out using the Interscan 1170 monitor (Interscan Corporation, Chatsworth, CA), 1 ppm full-scale deflection. Volunteers were asked to refrain from talking for 5 minutes prior to measurement. The monitor was zeroed on ambient air, and measurement performed by inserting a disposable ¼ " plastic straw approximately 4 cm into the oral cavity. The volunteer was asked to breathe through his/her nose during measurement. Results were recorded as peak ppb sulphide equivalents.

### Microbial counts

Unstimulated whole saliva was collected and diluted in saline. Plating was performed on blood agar. Plates were incubated aerobically at 37° C for 24 hours.

### Statistical evaluations

Comparison between the various rounds of the study was carried out using ANOVA or ANCOVA. Treatment effects were compared using the t-test with the Bonferroni correction.

### Results and Discussion:

Organoleptic assessments of whole mouth odor made by the three judges are summarized in Table II. In all cases, much larger reductions were observed in the scores of the experimental, as compared with control, subject groups. In the case of the experienced judge, the decrease in the scores of the active mouthwash group was highly significant as compared with placebo scores (p<0.0005, ANCOVA).

Similar results were obtained for odor deriving from the back of the tongue, and are shown in Table III.

**Table II**

| | **Baseline** | **90 Minutes** | **180 Minutes** |
|---|---|---|---|
| **Experimental group: experienced judge** | 1.9 +/- 0.9 | 1.2 +/- 1.07 | 1.0 +/- 1.23^{a} |
| **Control group: experienced judge** | 1.68 +/- 1.079 | 1.64 +/- 0.98 | 1.692 +/- 0.991 |
| **Experimental group: mean for three judges** | 2.14 /- 0.698 | 1.660 +/- 0.770 | 1.540 +/- 0.780 |
| **Control group: mean for three judges** | 1.71 +/- 0.714 | 1.583 +/- 0.775 | 1.577 +/- 0.799 |

| | | | |
|---|---|---|---|
| ^{a}Experimental group significantly different from control group (p=0.0308, t-test) | | | |

**Table III**

| | **Baseline** | **90 Minutes** | **180 Minutes** |
|---|---|---|---|
| **Experimental group: mean for 3 judges** | 2.400 +/- 0.707 | 1.958 +/- 0.834 | 1.593 +/- 0.649 |
| **Control group: mean for 3 judges** | 2.455 +/- 0.656 | 2.071 +/- 0.488 | 1.917 +/- 0.552 |
| **Experimental group: self assessment** | 1.740 +/- 1.347 | 1.750 +/- 1.445 | 2.240 +/- 1.091 |
| **Control group: self assessment** | 2.269 +/- 1.505 | 2.577 +/- 1.172 | 2.846 +/- 1.384 |

### Statistical significance:

- Judged results:: experimental group significantly different from control group (p = 0.0135; t-test);
- Self assessment:: experimental group significantly different from control group (p = 0.0304; t-test).

The results of the sulphide assay and of the microbial count measurements are shown in Table IV. The results for the sulphide assay demonstrate that for the expermental group, there was a significant decrease in salivary sulphide content at the 180 minute time point (p=0.0001; ANOVA). Similarly, the microbial count on blood agar was significantly reduced by the treatment at the 180 minute time point (p=0.0493; ANOVA).

**Table IV**

| **Tested** Parameter | **Baseline** | **90 minutes** | **180 minutes** |
|---|---|---|---|
| **Sulphide levels - Experimental** | 5.151+1- 0.234 | 4.915 +/- 0.095 | 4.825 +/-0.087 |
| **Sulphide levels - Control** | 5.060 +/- 0.245 | 5.043 +/- 0.205 | 4.971 +/- 0.276 |
| **Microbial counts - Experimental** | 7.001 +/- 0.926 | 5.996 +/- 0.919 | 6.804 +/- 1.203 |
| **Microbial counts -** Control | 6.231 +/- 0.827 | 6.184 +/- 0.825 | 6.771 +/- 0.873 |

### Example 3

### Inhibition of in vitro odor formation

A series of test tubes were prepared, each containing 5 ml of decarboxylase medium (0.5 % peptone, 0.3 % yeast extract, 0.1 % dextrose and 0.02 % bromocresol purple). To each tube was added 200 µl of freshly-taken morning saliva, and then 10 - 50 µl of a 1 % solution of a higher alcohol dissolved in 70 % ethanol or 100 % ethanol, as indicated. The tubes were incubated at 37° C for three days. Following incubation, the odor was determined by a panel of odor judges, using the following semi-quantitative scale
0 = no odor
1= very slight odor
2 = weak but noticeable odor
3 = medium odor
4 = foul odor
5 = extremely foul odor

The higher alcohols tested were alkanols of chain length C8, C9, C10, C11, C12, C14, C16 and C18. The C8 to C12 alcohols were prepared as 1 % solutions in 70 % ethanol, and the C14 to C18 alcohols were prepared as 1 % solutions in 100 % ethanol. Absence of alcoholic additives, 70 % ethanol alone, cetylpyridinium chloride (CPC) and chlorhexidine digluconate (CHX) were used as controls for the effect of the higher alcohols.

The results show that C9, C10 and C11 all cause inhibition of odor formation. The other alcohols tested are without effect in this model. The results are summarized in Table V below.

**Table V**

| | Odor Score (Judge 1) | Odor Score (Judge 2) |
|---|---|---|
| Control (no inhibitor) | 5 | 5 |
| 1% C8 50µl | 4 | 4 |
| 1% C8 40µl | 4 | 3 |
| 1% C8 30µl | 3 | 3 |
| 1% C8 20µl | 3 | 3 |
| 1% C8 10µl | 4 | 3 |
| 1% C9 50µl | 0 | 0 |
| 1% C9 40µl | 0 | 0 |
| 1% C9 30µl | 0 | 0 |
| 1% C9 20µl | 4 | 4 |
| 1% C9 10µl | 4 | 4 |
| 1% C10 50µl | 0 | 0 |
| 1% C10 40µl | 0 | 0 |
| 1% C10 30µl | 0 | 0 |
| 1% C10 20µl | 0 | 0 |
| 1% C10 10µl | 4 | 4 |
| 1% C11 50µl | 0 | 0 |
| 1% C11 40µl | 0 | 0 |
| 1% C11 30µl | 0 | 0 |
| 1% C11 20µl | 0 | 0.5 |
| 1% C11 10µl | 5 | 5 |
| 1% C12 50µl | 2 | 3.5 |
| 1% C12 40µl | 2 | 3 |
| 1% C12 30µl | 3 | 3 |
| 1% C12 20µl | 4 | 4 |
| 1% C12 10µl | 5 | 5 |
| 1% C14 50µl | 4 | 4 |
| 1% C14 40µl | 5 | 5 |
| 1% C14 30µl | 5 | 5 |
| 1% C14 20µl | 5 | 5 |
| 1% C14 10µl | 5 | 5 |
| 1% C16 50µl | 4 | 4 |
| 1% C16 40µl | 5 | 5 |
| 1% C16 30µl | 5 | 5 |
| 1% C16 20µl | 5 | 5 |
| 1% C16 10µl | 5 | 5 |
| 1% C18 50µl | 3.5 | 4 |
| 1% C18 40µl | 4 | 4 |
| 1% C18 30µl | 5 | 5 |
| 1% C18 20µl | 5 | 5 |
| 1% C18 10µl | 5 | 5 |
| 70% ethanol 200µl | 4 | 4.5 |
| 70% ethanol 100µl | 5 | 4.5 |
| 1% CPC 20µl | 0 | 0 |
| 1% CPC 10µl | 2 | 2.5 |
| 1% CPC 5µl | 4 | 4 |
| 1% CHX 40µl | 0 | 0 |
| 1% CHX 30µl | 0 | 0 |
| 1% CHX 20µl | 0 | 0 |
| 1% CHX 10µl | 2 | 1 |
| 1% CHX 5µl | 4 | 4.5 |

### Example 4

### In vitro specific antibacterial activity

The higher alcohols were tested for antimicrobial activity against Gram positive bacteria (*Streptococcus mutans*), Gram negative bacteria (*Pseudomonas* spp., *Escherichia coli, E. faecalis*) and the yeast *Candida albicans*. C8, C9, C10, C11 and C12 alcohols were prepared as 1 %, 0.1 % and 0.05 % (C9 only) solutions in 70 % ethanol. The C14, C16 and C18 alcohols were prepared as 1 % solutions in 100 % ethanol.

Five microliters of the higher alcohol solutions were placed on the surface of a rich (Brain-heart infusion) agar plate immediately after seeding with a mono-specific bacterial or yeast culture. The plates were incubated for 24 - 48 hours, and, following further microbial proliferation, the diameters of the zones of growth inhibition were measured (results expressed in centimeters).

The C9, C10, C11, C12 and C14 alcohols all inhibit microbial growth, the C9 alcohol having the widest spectrum of activity. The C16 and C18 alcohols cause moderate inhibition of *E. coli* (but not of *S. mutans*). C8 causes only partial inhibition of the yeast *C. albicans,* as evidenced by an opaque inhibition zone on the agar plate. These results are summarized in Table VI.

**Table VI**

| | *Pseudomonas aeruginosa* | *C. albicans* | *E. coli* | *E. faecalis* | *S. mutans* |
|---|---|---|---|---|---|
| 1% C8 | 0.5 | 0.7 +-² | 0.4+- | +- | - |
| 1% C9 | 0.6 | 0.9 | 1.0 | 0.9 | 0.9 |
| 1% C10 | 0.8 | 0.8 | 0.7 | 0.8+- | 0.8+- |
| 1% C11 | 0.7 | 0.8 | 0.7 | 0.9+- | 0.7+- |
| 1% C12 | +- | 0.8+- | 0.6+- | 0.7 | 0.6+- |
| 1% C14 | n.d.¹ | n.d. | 0.9 | n.d. | 1.0 |
| 1% C16 | n.d. | n.d. | 0.7 | n.d. | +- |
| 1% C18 | n.d. | n.d. | 0.8 | n.d. | +- |
| 0.1% C8 | n.d. | n.d. | 0.5+- | - | n.d. |
| 0.1% C9 | 0.7 | 0.8 | 1.0 | 0.7 | 0.9 |
| 0.1% C10 | 0.8 | 0.7 | 0.8 | 0.9 | n.d. |
| 0.1% C11 | 0.4 | 0.7 | 0.7 | 1.0 | n.d. |
| 0.1% C12 | +- | 0.6 | 0.6 | 1.0+ | n.d. |
| 0.05% C9 | 0.7 | 0.9+- | n.d. | n.d. | 0.7+- |
| 100%_{Ethanol} | n.d. | n.d. | 1.0 | n.d. | +- |
| 70% Ethanol | +- | n.d. | 0.4+- | - | - |

| | | | | | |
|---|---|---|---|---|---|
| ¹Not determined | | | | | |
| ²Partial inhibition (opaque inhibition zone) | | | | | |

In a further set of experiments, the effect of solvent type on the antimicrobial activity of the C9 alcohol was investigated. The following solvents were used to prepare 1% solutions of the C9 alcohol: water, 100% methanol, 100% glycerol, 100% 2-propanol, 70% ethanol. These solutions were tested for antimicrobial activity towards three microorganisms (*M. lysodeikticus, S. mutans* and *Corynebacterium xerosis*) using the same assay as described above.

The results (summarized in Table VII) show that when dissolved in water, C9 is almost devoid of activity. The solutions made in the lower alcohols, however, are highly active.

**Table VII**

| | *M. lysodeikticus* | *S. mutans* | *C. xerosis* |
|---|---|---|---|
| 1% C9 in water | - | - | 0.4+- |
| 1% C9 in 100% methanol | 0.9 | 0.9 | 0.4 |
| 100% methanol | 0.5+- | 0.6+- | 0.7 |
| 1% C9 in glycerol | +- | - | 0.4+- |
| 100% glycerol | - | - | 0.2 |
| 1% C9 in 2-propanol | 1.3 | 1.3 | 1.0 |
| 2-propanol | 1.1 | 0.9 | 0.8 |
| 1% C9 in 70% ethanol | 0.7 | 0.8 | 0.9 |
| 70% ethanol | +- | - | 0.5+- |

The relationship of the concentration of the C9 alcohol to its antimicrobial activity was also investigated. A series of solutions of the C9 alcohol was prepared in 70 % ethanol, with concentrations ranging from 0% (ethanol control) to 10%. These solutions were tested in the assay described above for their ability to inhibit the growth of the bacteria *M. lysodeikticus* and *S. mutans*.

The results are summarized in Table VIII, below. The antimicrobial activity of the C9 alcohol demonstrates only a weak concentration dependency. Over the range of concentrations tested, the greatest antibacterial activity was seen with C9 alcohol concentrations of 3% and greater. However, concentrations as low as 0.1 - 0.2 % are still highly active, causing more than 75 % of the maximal effect.

**Table VIII**

| Higher Alcohol Concentration | *M. lysodeikticus* | *S. mutans* |
|---|---|---|
| 10% C9 | 0.9 | 1.0 |
| 5% C9 | 0.9 | 0.9 |
| 4% C9 | 0.9 | 1.0 |
| 3% C9 | 0.9 | 0.9 |
| 2% C9 | 0.9 | 0.8 |
| 1% C9 | 0.7 | 0.7 |
| 0.5% C9 | 0.7 | 0.9 |
| 0.3% C9 | 0.7 | 0.8 |
| 0.2% C9 | 0.9 | 0.9+- |
| 0.1% C9 | 0.7 | 0.8+- |
| 0% C9 | - | - |

### Example 5

### Retentiveness of 1-nonanol in the oral cavity

1-nonanol was incorporated into a 2-phase (oil:aqueous) formulation having the following composition:

| | |
|---|---|
| sorbitol | 20 % |
| sodium saccharin | 0.05% |
| sodium benzoate | 0.1% |
| sorbic acid | 0.1% |
| vegetable oil | 15% |
| Breathanol | 0.5% |
| water | 64.25% |

About 400 microliters of the above composition was introduced into the mouth of a volunteer subject, using a pump spray. The composition was deposited at two sites: close to the tongue and at the entrance to the oro-pharynx. At regular intervals, the subject reported whether he could still detect the characteristic flavor of 1-nonanol. This flavor was still detected more than half-hour following administration of the spray. This indicates that 1-nonanol exhibits a high degree of retentiveness in the oral cavity, and thus is suitable for use on oral anti-odor and antimicrobial applications. The preparation was also deemed to be an effective anti-odor agent.

### Formulations

| **Formulation 1** | |
|---|---|
| **Toothpaste I** | |
| | % (w/w) |
| MIX A: | |
| Sodium alginate | 1 |
| Calcium carbonate | 38 |
| Aerosil 2000 silica (Degusse) | 1.6 |
| | |
| Glycerin (86 %) | 25 |
| Mineral oil DAB 10 | 0.5 |
| Sodium saccharin (Bayer) | 0.10 |
| Sodium monofluorophosphate (Phoskadent NA 211, Benckiser) | 0.76 |
| 1 % Breathanol™ | 0.02 - 2.5 |
| | |

| MIX B: | |
|---|---|
| Sodium lauryl sulfate | 1.5 |
| Nipagin M preservative (Nipa) | 0.1 |
| Water | to 100 % |

### Preparation:

1. The sodium alginate is added to the glycerin and allowed to swell.
2. The sodium lauryl sulfate is dissolved in approximately 5 parts water.
3. All the remaining ingredients are added.
4. The preparation is homogenized under vacuum.

To fully exploit the invention, the toothpaste should be brought into contact with the posterior region of the mouth by brushing the tongue, as well as with the teeth and gingivae, or alternatively by gargling.

| **Formulation 2** | |
|---|---|
| **Toothpaste II** | |
| | % (w/w) |
| Keitrol (Kelco) Xanthan gum | 0.8 |
| Glycerin (86 %) | 25 |
| Sorbitol (70 %) | 15 |
| Sident 12DS Silica (Degusse) | 21 |
| Syloblanc 34 Silica (Grece) | 1 |
| Titanium dioxide | 1 |
| Sodium fluoride | 0.22 |
| Sodium saccharin | 0.1 |
| Breathanol™ 1% | 0.02 - 2.5 |
| Sodium Lauryl sulfate | 2 |
| Preservative | 0.1 |
| Water | to 100 % |

### Preparation:

1. Swell the xanthan gum in the sorbitol and glycerin.
2. Add all other ingredients
3. Homogenize under vacuum.

To fully exploit the invention, the toothpaste should be brought into contact with the posterior region of the mouth by brushing the tongue, as well as with the teeth and gingivae, or alternatively by gargling.

| **Formulation 3** | |
|---|---|
| **Sugarless chewing gum** | |
| | % (w/w) |
| Gum base (Jagum T) | 30 |
| Sorbitol (70 %) | 14 |
| Glycerin | 1 |
| Sorbit powder | 40 |
| Palatinit | 9.8 |
| Mannitol | 3 |
| Xylitol | 2 |
| Aspartame | 0.1 |
| Acesulfam K | 0.1 |
| Breathanol™ 1% | 0.02 - 2.5 |

| **Formulation 4** | |
|---|---|
| **Hard candy** | |
| | % (w/w) |
| Saccharose | 57 |
| Glucose syrup | 29 |
| Breathanol™ 1% | 0.02 - 2.5 |
| Water | to 100 % |

### Preparation:

1. Dissolve the saccharose in the water at 110° C.
2. Add the glucose syrup and heat to 140° C.
3. Add the Breathanol™ and citric acid.
4. Pour into moulds at 130 - 135° C.

| **Formulation 5** | |
|---|---|
| **Soft candy** | |
| | % (w/w) |
| Sucrose/refined batch I | 35 |
| Spray-sour whey powder | 1.1 |
| Water | 10.5 |
| | |
| Glucose syrup (38-40 DE) | 42 |
| Hard fat D 700 S (SP 34-36° C) | 4.0 |
| | |
| Lecithin | 0.1 |
| | |
| Fudge mass | 5.8 |
| Gelatin (e.g., 230 Bloom) | 0.3 |
| Water (for swelling the gelatin) | 1.1 |
| | |
| Breathanol™ 1% | 0.02 - 2.5 |
| Water | to 100 % |

### Preparation:

1. Dissolve Sucrose and Whey powder in water and boil until clear
2. At about 115°C add glucose syrup and hard fat plus Lecithin and mix well
3. Boil the mass at 122°C.
4. Cool to about 90°C and then add fudge and well dissolved gelatin (at temperatures of more than 90°C the gelatin may be damaged)
   Disperse the breathanol well into the mass.
5. Cool the mass on a precooled cooling table, allow equilibration of temperature, and then stretch until the desired consistency is reached, shape into desired form.

| **Formulation 6** | |
|---|---|
| **Two-phase mouth-spray / mouthwash** | |
| | % (w/w) |
| 70 % sorbitol | 10 |
| Sodium benzoate | 0.1 |
| Sodium saccharine | 0.05 |
| Breathanol | 0.02 - 2.5 |
| Mint oil | 0.2 |
| Vegetable oil | 15.0 |
| Water | to 100 % |

To fully exploit the invention, the mouthwash should be gargled.

| **Formulation 7** | |
|---|---|
| **Single-phase mouth-spray / mouthwash** | |
| | % (w/w) |
| 70 % Sorbitol | 10 |
| Sodium benzoate | 0.1 |
| Sodium saccharine | 0.05 |
| Breathanol | 0.02 - 2.5 |
| Mint oil | 0.2 |
| Ethanol | 6 |
| Tagat RH40 (Tzifroni) | 2 |
| Water | to 100 % |

To fully exploit the invention, the mouthwash should be gargled.

| **Formulation 8** | |
|---|---|
| **Mouth drops** | |
| | % (w/w) |
| CMC | 3 |
| 70 % Sorbitol | 10 |
| Sodium benzoate | 0.1 |
| Sodium saccharine | 0.05 |
| Creamogen MZ (H&R) | 0.2 |
| Ethanol | 20 |
| Tagat RH40 (Tzifroni) | 2.1 |
| Breathanol | 0.2 - 2.5 |
| Mint oil | 0.2 |
| Water | to 100 % |

In addition to liquid and semi-solid products (such as e.g., candies), the compositions of the invention can be used to coat or impregnate inert materials, such as toothpicks, dental floss and the like. Furthermore, the compositions of the invention can be administered in gaseous form, e.g., they can be evaporated from cigarettes.

All the above description of preferred embodiments have been provided for the purpose of illustration and are not intended to limit the invention in any way. Many modifications can be made to the compositions and methods, without exceeding the scope of the invention.

## Claims

1. Use of a composition comprising a higher alcohol selected from 1-nonanol, 1-decanol and 1-undecanol, or mixtures thereof and taste-masking additives, as an oral anti-odor preparation.

2. Use according to claim 1, wherein the higher alcohol is 1-nonanol.

3. Use according to claim 1, wherein the higher alcohol is 1-decanol.

4. Use according to claim 1, wherein the higher alcohol is 1-undecanol.

5. Use according to any one of claims 1 to 4, wherein the taste-masking additives comprise nerol, citral and peppermint oil, or mixtures thereof.

6. An anti-odor toothpaste comprising a higher alcohol selected from 1-nonanol, 1-decanol and 1-undecanol, or mixtures thereof, together with one or more taste-masking additives.

7. An anti-odor toothpaste according to claim 6, wherein the higher alcohol is 1-nonanol.

8. An anti-odor toothpaste according to claim 6, wherein the higher alcohol is 1-decanol.

9. An anti-odor toothpaste according to claim 6, wherein the higher alcohol is 1-undecanol.

10. An anti-odor toothpaste according to any one of claims 6 to 9, wherein the taste-masking additives comprise nerol, citral and peppermint oil.

11. An anti-odor candy comprising a higher alcohol selected from 1-nonanol, 1-decanol and 1-undecanol, or mixtures thereof, together with one or more taste-masking additives.

12. An anti-odor candy according to claim 11, wherein the candy is a chocolate candy.

13. An anti-odor candy according to claim 11, wherein the candy is a chewable candy.

14. An anti-odor candy according to claim 11, wherein the candy is in the form of chewing gum.

15. A chewing gum according to claim 14, wherein the higher alcohol and the additives are incorporated in the gum, and the chewing gum is covered by a dragé.

16. An anti-odor candy according to claim 11, wherein the candy is a slowly dissolving product.

17. An anti-odor candy according to claim 16, wherein the slowly dissolving product is a pastille.

18. An anti-odor candy according to claim 16, wherein the slowly dissolving product is a hard candy.

19. An anti-odor candy according to any one of claims 11 to 18, wherein the higher alcohol is 1-nonanol.

20. An anti-odor candy according to any one of claims 11 to 18, wherein the higher alcohol is 1-decanol.

21. An anti-odor candy according to any one of claims 11 to 18, wherein the higher alcohol is 1-undecanol.

22. An anti-odor candy according to any one of claims 11 to 21, wherein the taste-masking additives comprise nerol, citral and peppermint oil.

23. An anti-odor mouthwash comprising a higher alcohol selected from 1-nonanol, 1-decanol and 1-undecanol, or mixtures thereof, together with taste-masking additives.

24. An anti-odor mouthwash according to claim 23, wherein the higher alcohol is 1-nonanol.

25. An anti-odor mouthwash according to claim 23, wherein the higher alcohol is 1-decanol.

26. An anti-odor mouthwash according to claim 23, wherein the higher alcohol is 1-undecanol.

27. An anti-odor mouthwash according to any one of claims 23 to 26, wherein the taste-masking additive comprises comprise nerol, citral and peppermint oil.

28. An anti-odor mouthspray comprising a higher alcohol selected from 1-nonanol, 1-decanol and 1-undecanol, or mixtures thereof, together with taste-masking additives.

29. An anti-odor cigarette, comprising a higher alcohol selected from 1-nonanol, 1-decanol and 1-undecanol, or mixtures thereof.

30. A toothpick coated or impregnated with a composition comprising a higher alcohol selected from among 1-nonanol, 1-decanol and 1-undecanol, or mixtures thereof, together with taste-masking additives.

31. A dental floss yarn coated or impregnated with a composition comprising a higher alcohol selected from among 1-nonanol, 1-decanol and 1-undecanol, or mixtures thereof, together with tasta-masking additives.

32. Use of an antimicrobially-effective amount of a higher alcohol selected from among 1-nonanol, 1-decanol and 1-undecanol, or mixtures thereof in a (C1-C4) alcohol or multi-phase oil-aqueous vehicle in the preparation of an antimicrobial composition suitable for oral use.

33. Use of a higher alcohol selected from among 1-nonanol, 1-decanol and 1-undecanol, or mixtures thereof, as an oral anti-odor agent.

34. Use according to claim 33, wherein the higher alcohol is dissolved in a two-phase aqueous/oil system.

35. Use according to claims 33 or 34, for removing or preventing odors.

36. A (C1-C4) alcohol supplemented with a higher alcohol selected from among 1-nonanol, 1-decanol and 1-undecanol, or mixtures thereof, for use as a synergistic antimicrobial composition.

37. Use according to claim 36, wherein the content of the higher alcohol is about 0.004% to 0.5%.

38. Use according to claim 36 or 87 wherein the (C1-C4) alcohol is selected from among ethanol, methanol and 2-propanol, or their mixtures.

39. Use according to claim 38 wherein the (C1-C4) alcohol is ethanol.

40. Use according to claim 32, wherein the antimicrobial composition is a wound-cleansing preparation, and wherein the higher alcohol is dissolved in a (C1-C4) alcohol medium.

41. A method for killing microorganisms, which comprises bringing the microorganiams to be killed into contact with a composition consisting of an antimicrobially-effective amount of a higher alcohol selected from among 1-nonanol, 1-decanol and 1-undecanol, or mixtures thereof, dissolved in a (C1-C4) alcohol medium.

42. A method according to claim 41 wherein the content of the higher alcohol is about 0.004% to 0.5%.

43. A method according to claim 41 or 42, wherein the (C1-C4) alcohol is selected from among ethanol, methanol and 2-propanol, or their mixtures.

44. A method according to claim 43, wherein the (C1-C4) alcohol is ethanol.

45. A method for increasing the anti-microbial activity of a (C1-C4) alcohol, or of a mixture of two or more (C1-C4) alcohols, comprising adding to said (C1-C4) alcohol or mixture of (C1-C4) alcohols, a synergistic amount of a higher alcohol, wherein the higher alcohol is selected from among 1-nonanol, 1-decanol and 1-undecanol, or mixtures thereof.

46. Use of a composition consisting of a (C1-C4) alcohol, and as an additive, a higher alcohol selected from among 1-nonanol, 1-decanol and 1-undecanol, or mixtures thereof, for the manufacture of a synergistic antimicrobial composition suitable for oral use.

47. Use of a composition comprising a higher alcohol selected from 1-nonanol, 1-decanol and 1-undecanol, or mixtures thereof for inhibiting or destroying oral gram negative bacteria.

48. Use of a composition comprising a higher alcohol selected from 1-nonanol, 1-decanol and 1-undecanol, or mixtures thereof for inhibiting or destroying oral malodor-causing bacteria.

49. An anti-odor composition adapted be brought into contact with the oro-pharynx and posterior portion of the oral cavity, comprising a higher alcohol selected from 1-nonanol, 1-decanol and 1-undecanol, or mixtures thereof, together with one or more taste-masking additives.

50. Anti-odor paste adapted to be applied to the tongue and to the teeth, comprising a higher alcohol selected from 1-nonanol, 1-decanol and 1-undecanol, or mixtures thereof, together with one or more taste-masking additives.

51. Anti-odor paste adapted to be supplied to the teeth and to be gargled, comprising a higher alcohol selected from 1-nonanol, 1-decanol and 1-undecanol, or mixtures thereof, together with one or more taste-masking additives.

## Patentansprüche

1. Verwendung einer Zusammensetzung enthaltend einen höheren Alkohol, der ausgewählt ist aus 1-Nonanol, 1-Decanol und 1-Undocanol oder Mischungen hiervon, und Geschmackmaskierungsadditive als orale Antiodorantzubereitung.

2. Verwendung nach Anspruch 1, worin der höhere Alkohol 1-Nonanol ist.

3. , Verwendung nach Anspruch 1, worin der höhere Alkohol 1-Decanol ist.

4. Verwendung nach Anspruch 1, worin der höhere Alkohol 1-Undecanol ist.

5. Verwendung nach einem der Ansprüche 1 bis 4, worin die Geschmackmaskierungsadditive Nerol, Citral und Pfefferminzöl oder Mischungen hiervon umfassen.

6. Antiodorantzahnpasta enthaltend einen höheren Alkohol, der ausgewählt ist aus 1-Nonanol, 1-Decanol und 1-Undecanol oder Mischungen hiervon, und ein oder mehr Geschmackmaskierungsadditive.

7. Antiodorantzahnpasta nach Anspruch 6, worin der höhere Alkohol 1-Nonanol ist.

8. Antiodorantzahnpasta nach Anspruch 6, worin der höhere Alkohol 1-Decanol ist.

9. Antiodorantzahnpasta nach Anspruch 6, worin der höhere Alkohol 1-Undecanol ist.

10. Antiodorantzahnpasta nach einem der Ansprüche 6 bis 9, worin die Geschmackmaskierungsadditive Nerol, Citral und Pfefferminzöl umfassen.

11. Antiodorantsüßware enthaltend einen höheren Alkohol, der ausgewählt ist aus 1-Nonanol, 1-Decanol und 1-Undecanol oder Mischungen hiervon, zusammen mit ein oder mehr Geschmackmaskierungsadditiven.

12. Antiodorantsüßware nach Anspruch 11, worin die Süßware eine Schokoladensüßware ist.

13. Antiodorantsüßware nach Anspruch 11, worin die Süßware eine Kausüßware ist.

14. Antiodorantsüßware nach Anspruch 11, worin die Süßware ein Kaugummi ist.

15. Kaugummi nach Anspruch 14, worin der höhere Alkohol und die Additive in den Gummi eingearbeitet sind und der Kaugummi mit einem Dragee überzogen ist.

16. Antiodorantsüßware nach Anspruch 11, worin die Süßware ein sich langsam auflösendes Produkt ist.

17. Antiodorantsüßware nach Anspruch 16, worin das sich langsam auflösende Produkt eine Pastille ist.

18. Antiodorantsüßware nach Anspruch 16, worin das sich langsam auflösende Produkt eine Hartsüßware ist.

19. Antiodorantsüßware nach einem der Ansprüche 11 bis 18, worin der höhere Alkohol 1-Nonanol ist.

20. Antiodorantsüßware nach einem der Ansprüche 11 bis 18, worin der höhere Alkohol 1-Decanol ist.

21. Antiodorantsüßware nach einem der Ansprüche 11 bis 18, worin der höhere Alkohol 1-Undecanol ist.

22. Antiodorantsüßware nach einem der Ansprüche 11 bis 21, worin die Geschmackmaskierungsadditive Norol, Citral und Pfefferminzöl umfassen.

23. Antiodorantmundwasser enthaltend oinen höheren Alkohol, der ausgewählt ist aus 1-Nonanol, 1-Decanol und 1-Undecanol oder Mischungen hiervon, und Geschmackmaskietungsadditive.

24. Antiodorantmundwasser nach Anspruch 23, worin der höhere Alkohol 1-Nonanol ist.

25. Antiodorantmundwasser nach Anspruch 23, worin der höhere Alkohol 1-Decanol ist.

26. , Antiodorantmundwasser nach Anspruch 23, worin der höhere Alkohol 1-Undecanol ist.

27. Antiodorantmundwasser nach einem der Ansprüche 23 bis 26, worin das Geschmackmaskierungsadditiv Nerol, Citral und Pfefferminzöl umfasst.

28. Antiodorantmundspray enthaltend einen höheren Alkohol, der ausgewählt ist aus 1-Nonanol, 1-Decanol und 1-Undecanol oder Mischungen hiervon, und Geschmackmaskierungsadditive.

29. Antiodorantzigarette enthaltend einen höheren Alkohol, der ausgewählt ist aus 1-Nonanol, 1-Decanol und 1-Undecanol oder Mischungen hiervon.

30. Zahnstocher, der mit einer Zusammensetzung beschichtet oder imprägniert ist, enthaltend einen höheren Alkohol, der ausgewählt ist aus 1-Nonanol, 1-Decanol und 1-Undecanol oder Mischungen hiervon, und Geschmackmaskierungsadditive.

31. Zahnflockgam, das mit einer Zusammensetzung beschichtet oder imprägniert ist, enthaltend einen höheren Alkohol, der ausgewählt ist aus 1-Nonanol, 1-Decanol und 1-Undecanol oder Mischungen hiervon, und Geschmackmaskierungsadditive.

32. Verwendung einer antimicrobiell wirksamen Menge eines höheren Alkohols, der ausgewählt ist aus 1-Nonanol, 1-Decanol und 1-Undecanol oder Mischungen hiervon, in einem C₁-C₄Alkohol oder einem mehrphasigen Träger aus Öl und Wasser zur Herstellung einer antimicrobiellen Zusammensetzung, die zur oralen Anwendung geeignet ist.

33. Verwendung eines höheren Alkohols ausgewählt aus 1-Nonanol, 1-Decanol und 1-Undecanol oder Mischungen hiervon als orales Antiodorant.

34. Verwendung nach Anspruch 33, worin der höhere Alkohol in einem Zweiphasensystem aus Wasser und Öl gelöst ist.

35. Verwendung nach Anspruch 33 oder 34 zur Entfernung oder Verhinderung von Gerüchen.

36. , C₁-C₄Alkohol, der mit einem höheren Alkohol ergänzt ist, ausgewählt aus 1-Nonanol, 1-Decanol und 1-Undecanol oder Mischungen hiervon, zur Verwendung als synergistische antimicrobielle Zusammensetzung.

37. Verwendung nach Anspruch 36, worin der Gehalt an höherem Alkohol etwa 0,004% bis 0,5% beträgt.

38. Verwendung nach Anspruch 36 oder 37, worin der C₁-C₄Alkohol ausgewählt ist aus Ethanol, Methanol und 2-Propanol oder Mischungen hiervon.

39. Verwendung nach Anspruch 38, worin der C₁-C₄Alkohol Ethanol ist.

40. Verwendung nach Anspruch 32, worin die antimicrobielle Zusammensetzung eine Wundreinigungszubereitung ist und worin der höhere Alkohol in einem C₁-C₄Alkoholmedium gelöst ist.

41. Verfahren zur Abtötung von Microorganismen, umfassend ein Inkontaktbringen der zu tötenden Microorganismen mit einer Zusammensetzung, die aus einer antimicrobiell wirksamen Menge eines höheren Alkohols besteht, der ausgewählt ist aus 1-Nonanol, 1-Decanol und 1-Undecanol oder Mischungen hiervon, und gelöst ist in einem C₁-C₄Alkoholmedium.

42. Verfahren nach Anspruch 41, worin der Gehalt an höherem Alkohol etwa 0,004% bis 0,5% beträgt.

43. Verfahren nach Anspruch 41 oder 42, worin der C₁-C₄Alkohol ausgewählt ist aus Ethanol, Methanol und 2-Propanol oder Mischungen hiervon.

44. Verfahren nach Anspruch 43, worin der C₁-C₄Alkohol Ethanol ist.

45. Verfahren zur Brhöhung der antimicrobiellen Wirksamkeit eines C₁-C₄Alkohols oder einer Mischung aus zwei oder mehr C₁-C₄Alkoholen, wobei der C₁-C₄Alkohol oder die Mischung aus C₁-C₄Alkoholen mit einer synergistischen Menge eines höheren Alkohols versetzt wird, der ausgewählt ist aus 1-Nonanol, 1-Decanol und 1-Undecanol oder Mischungen hiervon.

46. Verwendung einer Zusammensetzung bestehend aus einem C₁-C₄Alkohol und einem höheren Alkohol, ausgewählt aus 1-Nonanol, 1-Dacanol und 1-Undecanol oder Mischungen hiervon, als Additiv zur Herstellung einer synergistischen antimicrobiellen Zusammensetzung, die zur oralen Anwendung geeignet ist.

47. Verwendung einer Zusammensetzung enthaltend einen höheren Alkohol, der ausgewählt ist aus 1-Nonanol, 1-Decanol und 1-Undecanol oder Mischungen hiervon, zur Hemmung oder Zerstörung oraler gramnegativer Bakterien.

48. Verwendung einer Zusammensetzung enthaltend einen höheren Alkohol, der ausgewählt ist aus 1-Nonanol, 1-Decanol und 1-Undecanol oder Mischungen hiervon, zur Hemmung oder Zerstörung oraler und einen schlechten Geruch verursachender Bakterien.

49. Antiodorantzusammensetzung, die für eine Inkontaktboringung mit dem Oropharynx und dem hinteren Teil der Oralkavität angepasst ist, enthaltend einen höheren Alkohol, der ausgewählt ist aus 1-Nonanol, 1-Decanol und 1-Undecanol oder Mischungen hiervon, und ein oder mehr Geschmackmaskierungsadditive.

50. Antiodorantpaste, dio zur Anwendung auf der Zunge und den Zahnen angepasst ist, enthaltend einen höheren Alkohol, der ausgewählt ist aus 1-Nonanol, 1-Decanol und 1-Undecanol oder Mischungen hiervon, und ein oder mehr Geschmackmaskierungsadditive.

51. Antiodorantpaste, die für eine Versorgung der Zähne oder zum Gurgeln angepasst ist, enthaltend einen höheren Alkohol, der ausgewählt ist aus 1-Nonanol, 1-Decanol und 1-Undecanol oder Mischungen hiervon, und ein oder mehr Geschmackmaskierungsadditive.

## Revendications

1. Utilisation d'une composition comprenant un alcool à haut poids moléculaire choisi parmi les 1-nonanol, 1-décanol et 1-undécanol, ou des mélanges de ceux-ci et des additifs masquant le goût, en tant que préparation orale anti-odeur.

2. Utilisation selon la revendication 1, dans laquelle l'alcool à haut poids moléculaire est le 1-nonanol.

3. Utilisation selon la revendication 1, dans laquelle l'alcool à haut poids moléculaire est le 1-décanol.

4. Utilisation selon la revendication 1, dans laquelle l'alcool à haut poids moléculaire est le 1-undécanol.

5. Utilisation selon l'une quelconque des revendications 1 à 4, dans laquelle les additifs masquant le goût comprennent une huile de nérol, de citral et de menthe poivrée, ou des mélanges de ceux-ci.

6. Dentifrice anti-odeur comprenant un alcool à haut poids moléculaire choisi parmi les 1-nonanol, 1-décanol et 1-undécanol, ou des mélanges de ceux-ci, ensemble avec un ou plusieurs additifs masquant le goût.

7. Dentifrice anti-odeur selon la revendication 6, dans lequel l'alcool à haut poids moléculaire est le 1-nonanol.

8. Dentifrice anti-odeur selon la revendication 6, dans lequel l'alcool à haut poids moléculaire est le 1-décanol.

9. Dentifrice anti-odeur selon la revendication 6, dans lequel l'alcool à haut poids moléculaire estl-undécanol.

10. Dentifrice anti-odour selon l'une quelconque des revendications 6 à 9, dans lequel les additifs masquant le goût comprennent une huile de nérol, de citral et de menthe poivrée.

11. Bonbon anti-odeur comprenant un alcool à haut poids moléculaire choisi parmi les 1-nonanol, 1-décanol et 1-undécanol, ou des mélanges de ceux-ci, ensemble avec un ou plusieurs additifs masquant le goût.

12. Bonbon anti-odeur selon la revendication 11, dans lequel le bonbon est un bonbon au chocolat.

13. Bonbon anti-odeur selon la revendication 11, dans lequel le bonbon est un bonbon croquable.

14. Bonbon anti-odeur selon la revendication 11, dans lequel le bonbon est sous la forme de gomme à mâcher.

15. Gomme à mâcher selon la revendication 14, dans laquelle l'alcool à haut poids moléculaire et les additifs sont incorporés dans la gomme, et la gomme à mâcher est couverte par une dragée.

16. Bonbon anti-odeur selon la revendication 11, dans lequel le bonbon est un produit se dissolvant lentement.

17. Bonbon anti-odeur selon la revendication 16, dans lequel le produit se dissolvant lentement est une pastille.

18. Bonbon anti-odeur selon la revendication 16, dans lequel le produit se dissolvant lentement est un bonbon dur.

19. Bonbon anti-odeur selon l'une quelconque des revendications 11 à 18, dans lequel l'alcool à haut poids moléculaire est le 1-nonanol.

20. Bonbon anti-odeur selon l'une quelconque des revendications 11 à 18, dans lequel l'alcool à haut poids moléculaire est le 1-décanol.

21. Bonbon anti-odeur selon l'une quelconque des revendications 11 à 18, dans lequel l'alcool à haut poids moléculaire est le 1-undécanol.

22. Bonbon anti-odeur selon l'une quelconque des revendications 11 à 21, dans lequel les additifs masquant le goût comprennent une huile de nérol, de citral et de menthe poivrée.

23. Bain de bouche anti-odeur comprenant un alcool à haut poids moléculaire choisi parmi les 1-nonanol, 1-décanol et 1-undécanol, ou des mélanges de ceux-ci, ensemble avec des additifs masquant le goût.

24. Bain de bouche anti-odeur selon la revendication 23, dans lequel l'alcool à haut poids moléculaire est le 1-nonanol.

25. Bain de bouche anti-odeur selon la revendication 23, dans lequel l'alcool à haut poids moléculaire est 1-décanol.

26. Bain de bouche anti-odeur selon la revendication 23, dans lequel l'alcool à haut poids moléculaire est 1-undécanol,

27. Bain de bouche anti-odeur selon l'une quelconque des revendications 23 à 26, dans lequel l'additif masquant le goût comprend une huile de nérol, de citral et de menthe poivrée.

28. Aérosol buccal anti-odeur comprenant un alcool à haut poids moléculaire choisi parmi les 1-nonanol, 1-décanol et 1-undécanol, ou des mélanges de ceux-ci, ensemble avec des additifs masquant le goût.

29. Cigarette anti-odeur comprenant un alcool à haut poids moléculaire choisi parmi les 1-nonanol, 1-décanol et 1-undécanol, ou des mélanges de ceux-ci.

30. Cure-dent revêtu ou imprégné avec une composition comprenant un alcool à haut poids moléculaire choisi parmi les 1-nonanol, 1-décanol et 1-undécanol, ou des mélanges de ceux-ci, ensemble avec des additifs masquant le goût.

31. Fil de soie dentaire revêtu ou imprégné avec une composition comprenant un alcool à haut poids moléculaire choisi parmi les 1-nonanol, 1-décanol et 1-undécanol, ou des mélanges de ceux-ci, ensemble avec des additifs masquant le goût.

32. Utilisation d'une quantité efficace de manière antimicrobienne d'un alcool à haut poids moléculaire choisi parmi les 1-nonanol, 1-décanol et 1-undécanol, ou des mélanges de ceux-ci dans un véhicule d'alcool en C₁-C₄ ou huile-aqueux multi-phases pour la préparation d'une composition antimicrobienne appropriée pour une utilisation orale.

33. Utilisation d'un alcool à haut poids moléculaire choisi parmi les 1-nonanol, 1-décanol et 1-undécanol, ou des mélanges de ceux-ci, en tant qu'agent anti-odeur oral.

34. Utilisation selon la revendication 33, dans laquelle l'alcool à haut poids moléculaire est dissous dans un système aqueux/huile à deux phases.

35. Utilisation selon les revendications 33 ou 34, pour supprimer ou empêcher les odeurs.

36. Alcool en C₁-C₄ supplémenté avec un alcool à haut poids moléculaire choisi parmi les 1-nonanol, 1-décanol et 1-undécanol, ou des mélanges de ceux-ci, pour une utilisation en tant que composition antimicrobienne synergiste.

37. Utilisation selon la revendication 36, dans laquelle la teneur de l'alcool à haut poids moléculaire est environ de 0,004 % à 0,5 %.

38. Utilisation selon la revendication 36 ou 37, dans laquelle l'alcool en C₁-C₄ est choisi parmi les éthanol, méthanol et 2-propanol, ou leurs mélanges.

39. Utilisation selon la revendication 38, dans laquelle l'alcool en C₁-C₄ est l'éthanol.

40. Utilisation selon la revendication 32, dans laquelle la composition antimicrobienne est une préparation de nettoyage de plaies, et dans laquelle l'alcool à haut poids moléculaire est dissous dans un milieu d'alcool en C₁-C₄.

41. Procédé pour tuer des microorganismes, qui comprend la mise en contact des microorganismes à tuer avec une composition constituée d'une quantité efficace de manière antimicrobienne d'un alcool à haut poids moléculaire choisi parmi les 1-nonanol, 1-décanol et 1-undécanol, ou des mélanges de ceux-ci, dissous dans un milieu d'alcool en C₁-C₄.

42. Procédé selon la revendication 41, dans lequel la teneur de l'alcool à haut poids moléculaire est environ de 0,004 % à 0,5 %.

43. Procédé selon la revendication 41 ou 42, dans lequel l'alcool en C₁-C₄ est choisi parmi les éthanol, méthanol et 2-propanol, ou leurs mélanges.

44. Procédé selon la revendication 43, dans lequel l'alcool en C₁-C₄ est l'éthanol.

45. Procédé pour augmenter l'activité antimicrobienne d'un alcool en C₁-C₄, ou d'un mélange de deux ou plusieurs alcools en C₁-C₄, comprenant l'addition audit alcool en C₁-C₄ ou mélange d'alcools en C₁-C₄, d'une quantité synergiste d'un alcool à haut poids moléculaire, dans lequel l'alcool à haut poids moléculaire est choisi parmi les 1-nonanol, 1-décanol et 1-undécanol, ou des mélanges de ceux-ci.

46. Utilisation d'une composition constituée d'un alcool en C₁-C₄, et en tant qu'un additif, un alcool à haut poids moléculaire choisi parmi les 1-nonanol, 1-décanol et 1-undécanol, ou des mélanges de ceux-ci pour la production d'une composition antimicrobienne synergiste appropriée pour une utilisation orale.

47. Utilisation d'une composition comprenant un alcool à haut poids moléculaire choisi parmi les 1-nonanol, 1-décanol et 1-undécanol, ou des mélanges de ceux-ci pour inhiber ou détruire les bactéries gram-négatives orales.

48. Utilisation d'une composition comprenant un alcool à haut poids moléculaire choisi parmi les 1-nonanol, 1-décanol et 1-undécanol, ou des mélanges de ceux-ci pour inhiber ou détruire les bactéries causant de mauvaises odours.

49. Composition anti-odeur conçue pour être mise en contact avec la partie oro-pharynx et postérieure de la cavité orale, comprenant un alcool à haut poids moléculaire choisi parmi les 1-nonanol, 1-décanol et 1-undécanol, ou des mélanges de ceux-ci, ensemble avec un ou plusieurs additifs masquant le goût.

50. Pâte anti-odeur conçue pour être appliquée sur la langue et sur les dents, comprenant un alcool à haut poids moléculaire choisi parmi les 1-nonanol, 1-décanol et 1-undécanol, ou des mélanges de ceux-ci, ensemble avec un ou plusieurs additifs masquant le goût.

51. Pâte anti-odeur conçue pour être adaptée aux dents et pour se gargariser, comprenant un alcool à haut poids moléculaire choisi parmi les 1-nonanol, 1-décanol et 1-undécanol, ou des mélanges de ceux-ci, ensemble avec un ou plusieurs additifs masquant le goût.
